**Europäisches Patentamt**

**European Patent Office**

**Office européen des brevets**

(19)

(11) Veröffentlichungsnummer: **0 242 529**
**A2**

(12) # EUROPÄISCHE PATENTANMELDUNG

(21) Anmeldenummer: **87102338.8**

(22) Anmeldetag: **19.02.87**

(51) Int. Cl.⁴: **C08F 251/00 , A61L 15/00**

(30) Priorität: **19.04.86 DE 3613309**

(43) Veröffentlichungstag der Anmeldung:
**28.10.87 Patentblatt 87/44**

(84) Benannte Vertragsstaaten:
**AT BE CH DE ES FR GB IT LI NL SE**

(71) Anmelder: **HÜLS AKTIENGESELLSCHAFT
- RSP Patente / PB 15 - Postfach 13 20
D-4370 Marl 1(DE)**

(72) Erfinder: **Heidel, Klaus, Dr.
Veilchenstrasse 10
D-4370 Marl(DE)**

(54) **Verfahren zur Herstellung von wasserabsorbierenden und wasserquellfähigen Polysaccharidpfropfpolymeren.**

(57) Die Erfindung betrifft ein einstufiges Verfahren zur Herstellung von Polysaccharidpfropfpolymeren. Durch Einsatz eines nichtionischen, lipophilen und eines nichtionischen, hydrophilen Tensids gelingt es, feinteilige Pfropfpolymere mit sehr hoher Wasseraufnahmefähigkeit herzustellen.

EP 0 242 529 A2

## Verfahren zur Herstellung von wasserabsorbierenden und wasserquellfähigen Polysaccharidpfropfpolymeren

Die Erfindung betrifft ein verbessertes Verfahren zur Herstellung von stark wasserabsorbierenden und wasserquellfähigen Pfropfpolymeren aus einem Polysaccharid als Pfropfgrundlage und wasserlöslichen, ethylenisch ungesättigten, Carboxylgruppen enthaltenden Monomeren.

Wasserabsorbierende Polymere finden vielfältige Verwendung auf dem Sanitär-und Hygienesektor als Wasserabsorptionsmittel in Papierwindeln und -tüchern, als Tampons, Krankenunterlagen, Elektrolytverdickern in Trockenbatterien, als Feuchthaltemittel oder Wasserspeicher in der Landwirtschaft und als Trocknungsmittel.

Geeignete Polymere sind derivatisierte, meist mit wasserlöslichen Vinylmonomeren gepfropfte Polysaccharide wie Carboxymethylcellulose, hydrolysierte Stärke-Acrylnitril-Pfropfpolymere, Acrylsäure-Stärke-Pfropfpolymere oder vollsynthetische, schwach vernetzte Polymere wie vernetztes Polyethylenoxid oder teilvernetzte Polyacrylsäuresalze. Von diesen Polymeren weisen Carboxymethylcellulose und teilvernetztes Polyethylenoxid jedoch nur eine Wasseraufnahmekapazität von etwa 30 g vollentsalztem Wasser (VE-Wasser)/g Polymer auf.

Teilverseifte Stärke-Acrylnitril-Pfropfpolymere haben eine hohe Wasseraufnahmefähigkeit von 300 bis 500 g VE-Wasser/g. Man kann diese Polymere durch Pfropfung von Acrylnitril auf Stärke in wäßriger Suspension in Gegenwart von Cer(IV)-Salzen, nachfolgende Hydrolyse durch wäßrige Natron-oder Kalilauge und gegebenenfalls Reinigung durch Umfällung herstellen. Bei diesem Verfahren wird Acrylnitril, ein als gefährlich eingestufter Arbeitsstoff, gehandhabt. Außerdem müssen die Produkte, besonders wenn sie in den Hygienesektor gehen sollen, durch Entgasung vollständig von Monomeren befreit werden. Ferner ist die Hydrolyse der Nitrilgruppen ein technisch schwieriger Prozeß, da dabei hochviskose, wassergequollene Rohprodukte mit teigähnlicher Konsistenz und verschlechterter Wasserabsorption entstehen. Durch umständliche Reinigungsschritte kann die Wasserabsorptionsfähigkeit wieder verbessert werden.

Es ist daher schon versucht worden, wasserabsorptionsfähige Pfropfpolymere durch direktes Aufpfropfen von wasserlöslichen Monomeren wie Acrylsäure und Acrylamid auf Stärke in wäßriger oder alkoholischer Lösung durchzuführen.

Die japanische Patentschrift 40-43 408 beschreibt ein Verfahren zur Pfropfung von ungesättigten wasserlöslichen Monomeren wie Acrylsäure auf gelatinierte Stärke in wäßriger Lösung in Gegenwart eines wasserlöslichen Initiierungssystems aus Wasserstoffperoxid und Ascorbinsäure. Dabei werden wasserlösliche Polymere mit hoher Eindickwirkung, aber geringer Wasserabsorptionsfähigkeit von etwa 100 g VE-Wasser/g erhalten.

Nach der japanischen Patentschrift 80-139 408 ist ferner bekannt, daß durch Pfropfung von Stärke mit (Meth)Acrylsäure in wäßriger Lösung und Initiierung mit wasserlöslichen Peroxiden nur dann gelartige wasserabsorbierende Polymere mit guter Wasserabsorptionsfähigkeit von über 200 g VE-Wasser/g erhalten werden, wenn sie durch Einbau kleiner Anteile von di-oder trifunktionellen Verbindungen wie Glycerindiglycidylether, N.N'-Methylenbisacrylamid oder Trimethylglykolpropantriacrylat schwach vernetzt werden.

Bei der vernetzenden Polymerisation von wasserlöslichen Monomeren in wäßriger Lösung zur Herstellung von teilchenförmigen Gelen ergeben sich folgende Schwierigkeiten:

1) Die vernetzende Polymerisation führt prinzipiell zu einem Netzwerk von wenig elastischen Ketten.

2) Man erhält als Reaktionsprodukte wassergequollene Gele, die schwierig zu handhaben sind. Der Feststoff muß durch Fällung von der Wasserphase abgetrennt werden, wobei er in verklumpter Form anfällt. Er muß dann getrocknet und gemahlen werden.

3) Eine Durchmischung und Rührung von wäßrigen Lösungen ist wegen der Quellung der Pfropfpolymeren und einer hohen Viskosität auch bei niedrigen Feststoffgehalten von 10 bis 20 % nicht möglich. Deshalb ist eine kontrollierte Reaktion in üblichen Rührapparaturen nicht durchführbar.

4) Ein effektives Aufpfropfen von wasserlöslichen Monomeren auf Stärke unter Bildung von gelhaltigen Pfropfpolymeren mit einer für gute Wasserabsorptionsfähigkeit erforderlichen verzweigtkettigen Struktur ist in rein wäßrigen Reaktionsmedien nicht möglich. Denn wasserlösliche Monomere wie Acrylsäure oder Acrylamid polymerisieren hier in einer unvermeidbaren Nebenreaktion zu wasserlöslichen, nicht gepfropften Homopolymeren, die lediglich eindickend wirken, nicht aber wasserquellbar sind und deshalb die Wasserabsorptionsfähigkeit vermindern.

Bei dieser wäßrigen Lösungspolymerisation sind Maßnahmen zur Änderung des Pfropfgrades wie der Erhöhung der Konzentration der Pfropfgrundlage, der Monomerkonzentration und der Initiatorkonzentration nur in sehr beschränktem Umfang durchführbar. Eine Konzentrationserhöhung der Pfropfgrundlage führt zu einer weiteren Viskositätserhöhung. Eine Erhöhung der Initiatorkonzentration kann zu einer unbeherrschbar hohen Polymerisationsgeschwindigkeit und zu niedrigem Molekulargewicht führen.

Als verbessertes Polymerisationsverfahren zur Herstellung hochmolekularer Homo-und Pfropfpolymerer in fester feinteiliger Form ist die Wasser-in-Öl-Suspensionspolymerisation (inverse Suspensionspolymerisation) entwickelt worden.

Dieses Verfahren hat sich als sehr vorteilhaft zur Herstellung von Pfropfpolymeren aus Polysacchariden wie Stärke und wasserlöslichen Monomeren erwiesen, da hiermit die Voraussetzung für eine effektive Pfropfung wie hohe Konzentration von Stärke, Monomeren und Peroxid bei guter Rührfähigkeit der Suspensionen erfüllt werden. In DE-PS 28 40 010 wird die inverse Suspensionspolymerisation zur Pfropfung von Polysacchariden mit wasserlöslichen Monomeren beschrieben. Dabei wird eine halbkontinuierliche Suspensionspolymerisation durchgeführt. In Petrolether, das ein lösemittellösliches, grenzflächenaktives Mittel wie Sorbitanmonooleat enthält, wird Stärke als Pfropfsubstrat suspendiert. Dazu wird eine wäßrige Lösung des Monomers, die in einem gesonderten Behälter bereitet wird, zugegeben und die Polymerisation durch Zusatz eines radikalischen Initiators gestartet und über 0.5 bis 6 Stunden fortgeführt. Das gebildete Pfropfmischpolymerisat wird in Form fester Teilchen isoliert. Das grenzflächenaktive Mittel stellt einen integrierten Bestandteil des Verfahrens dar.

Das Verfahren hat folgende Eigenschaften:

1. Die Monomeren werden in Form einer wäßrigen Lösung, die in einem gesonderten Reaktionsgefäß bereitet wird, zusammen mit einer relativ hohen Zusatzmenge eines wasserlöslichen Tensids, zur organischen Phase zugegeben, wobei das Tensid für eine homogene Verteilung der wäßrigen Phase in der organischen Phase als notwendig angesehen wird. Diese sogenannte halbkontinuierliche Verfahrensmethode erfordert somit ein zusätzliches Reaktionsgefäß, das beim Einsatz von Acrylsäure als Monomer mit einer Kühleinrichtung zur Abführung der Neutralisationswärme ausgerüstet sein muß.

2. Es werden vorzugsweise Gemische von ionischen und nichtionischen Tensiden in hohen Zusatzmengen von 0.5 bis 12 %, bezogen auf das Lösungsmittel, eingesetzt.

In der japanischen Patentschrift 80-161 813 wird die Herstellung eines wasserabsorbierenden Pfropfpolymeren durch Pfropfung eines Polysaccharids mit Acrylsäure in einem Benzin-Kohlenwasserstoff als Lösemittel in Gegenwart eines organophilen nichtionischen Tensids und eines wasserlöslichen Initiators beschrieben. Beispielsweise wird in einer organischen Phase aus n-Hexan mit einem Zusatz von 2 % Sorbitanmonostearat eine wäßrige Phase aus Kornstärke, Acrylsäure, Natronlauge und Persulfat dispergiert und bei 60 °C in 6 Stunden zu einem gelartigen Pfropfpolymerisat mit einem Wasseraufnahmevermögen von 600 g VE-Wasser/g polymerisiert.

In EP 0 036 463 A2, DE-OS 33 31 644 und DE-OS 35 07 775 werden Verfahren der inversen Suspensionspolymerisation von Carboxyl-oder Carboxylatgruppen enthaltenden wasserlöslichen Monomeren in Gegenwart kleiner Zusatzmengen eines Quervernetzungsmittels zur Herstellung von stark wasserabsorbierenden, gelartigen Polymerisaten beschrieben. Die Polymerisation wird in allen Beispielen nach der halbkontinuierlichen Verfahrensweise mit relativ hohem apparativen Aufwand durchgeführt, wobei die wäßrige Monomerlösung durch Neutralisation von Acrylsäure mit einer wäßrigen Alkali-oder Ammoniumhydroxid-Lösung unter Kühlung in einem gesonderten Reaktionsgefäß hergestellt und zur organischen Phase dosiert wird. Diese Verfahren unterscheiden sich durch die Art der verwendeten Tenside, wobei man zur Charakterisierung der Tenside das Hydrophil-Lipophil-Gleichgewicht (HLB-Wert) heranzieht. Lipophile Tenside weisen einen HLB-Wert von 0 bis 10 und hydrophile Tenside einen HLB-Wert von 10 bis 20 auf.

In EP 0 036 463 A2 wird ein öllöslicher oberflächenaktiver Stoff mit relativ hohem HLB-Wert von 8 bis 12 eingesetzt, wobei Sorbitanmonolaurat bevorzugt wird. Bei der Polymerisation tritt starke Gel-und Klumpenbildung auf. Die Produkte zeichnen sich durch ein hohes Wasseraufnahmevermögen von teilweise etwa 1 000 g VE-Wasser/g aus.

In DE-OS 33 31 644 und DE-OS 35 07 775 wird die wäßrige Monomerlösung zur heißen organischen Phase, die ein öllösliches Tensid mit niedrigem HLB-Wert enthält, zudosiert. Die Wasserabsorptionskapazitäten der hier hergestellten Polymeren liegen bei 450 bis 750 g VE-Wasser/g.

Diesen Patentschriften ist zu entnehmen, daß bei halbkontinuierlicher inverser Suspensionspolymerisation bei Verwendung von nichtionischen Tensiden mit hohem HLB-Wert, also mit hoher Wasserlöslichkeit, großteilige, stark zur Vergelung neigende Polymere mit hoher Wasserquellbarkeit entstehen, während bei Verwendung nichtionischer Tenside mit niedrigem HLB-Wert feinteilige, wenig zu Verklumpung neigende Polymere mit niedriger Wasseraufnahmefähigkeit erhalten werden.

Aufgabe der vorliegenden Erfindung ist es, ein vereinfachtes Verfahren zur Herstellung von feinteiligen und gleichzeitig stark wasserquellbaren aber wenig wasserlöslichen Pfropfpolymeren aus Polysacchariden und Carboxyl-oder Carboxylatgruppen enthaltenden Vinylmonomeren bereitzustellen. Dabei sollen bei der Polymerisation die Pfropfpolymere direkt als Pulver mit hoher Wasserabsorptionskapazität anfallen und Wandabscheidungen und Gelklumpenbildung weitgehend unterdrückt werden.

Die Aufgabe wird überraschenderweise dadurch gelöst, daß man eine einstufige, diskontinuierliche, radikalische Polymerisation in einer Dispersion aus

-einem aliphatischen Kohlenwasserstoff,

-einer wäßrigen Lösung mindestens eines wasserlöslichen, ethylenisch ungesättigten Monomeren,

-einem Polysaccharid,

-einem nichtionischen, wasserunlöslichen, im aliphatischen Kohlenwasserstoff mindestens teilweise löslichen Tensid mit einem HLB-Wert kleiner als 10 und

-einem nichtionischen, wasserlöslichen, Polyethylenglykolgruppen enthaltenden Tensid mit einem HLB-Wert größer als 10

bei 40 bis 100 °C durchführt. Das gebildete Pfropfpolymer wird nach der Reaktion als Pulver abgetrennt.

Im Gegensatz zum halbkontinuierlichen Verfahren wird bei diesem einstufigen, diskontinuierlichen, inversen Suspensionspolymerisationsverfahren (Meth)Acrylsäure im Polymerisationskessel in-situ in Gegenwart der organischen Phase in der wäßrigen Phase durch eine wäßrige Lauge neutralisiert. Man kann dabei (Meth)Acrylsäure vorlegen und Lauge zudosieren oder Lauge vorlegen und (Meth)Acrylsäure zugeben.

Der Kohlenwasserstoff kann 6 bis 12 C-Atome aufweisen. Man kann aliphatische oder alicyclische Kohlenwasserstoffe wie Cyclohexan, n-Hexan, $C_8$-Isoparaffine oder technische Benzinfraktionen wie Normalbenzin, Ligroin, Testbenzin oder Solventnaphtha mit einem Aromatenanteil bis zu 20 % und einen Siedepunkt im Bereich von 50 bis 200 °C verwenden.

Die wasserlöslichen Monomeren bestehen zu 50 bis 100 Gew.-% aus Acrylsäure oder Methacrylsäure. Daneben können bis zu 50 Gew.-% wasserlösliche Comonomere wie Acrylamid, Methacrylamid, Na-Salz von 2-Acrylamido-2-methylpropansulfonsäure, 2-Methacryloylethansulfonsäure, 2-Hydroxyethylacrylat, 2-Hydroxyethylmethacrylat, N,N-Dimethylaminoethylacrylat oder -methacrylat oder dessen quaternäre Ammoniumsalze eingesetzt werden.

Freie Carboxylgruppen enthaltenden Monomeren werden im Polymerisationskessel durch Zugabe von wäßrigen Lösungen aus Natrium-, Kalium-oder Ammoniumhydroxid zu 50 bis 95 Mol-%, vorzugsweise zu 65 bis 80 Mol-% teilneutralisiert. Ist der Neutralisationsgrad kleiner als 50 Mol-%, nimmt die Wasseraufnahmefähigkeit ab, liegt er höher als 95 %, werden keine wasserquellbaren, sondern wasserlösliche Pfropfpolymeren erhalten. Es werden zweckmäßigerweise Alkali-oder Ammoniumhydroxid-Lösungen mit einer Konzentration von 15 bis 30 Gew.-% verwendet.

Nach der Bereitung der wäßrigen Phase und Homogenisierung durch Rühren erfolgt die Zugabe des Polysaccharids in körniger Form. Geeignet sind native Stärken aus Kartoffeln, Mais, Weizen, Reis oder Tapiocawurzeln, ferner Wachsmais oder High-Amylose-Stärke sowie deren Derivate, insbesondere Stärkeether und -ester. Geeignet sind auch Cellulose oder Cellulosederivate.

Polysaccharid und wasserlösliches Monomer werden in solchen Gewichtsverhältnissen eingesetzt, daß Pfropfpolymere mit 10 bis 70 Gew.-% Polysaccharid und 90 bis 30 Gew.-% Vinylpolymer erhalten werden. Vorzugsweise besteht das Pfropfpolymer aus 30 bis 50 Gew.-% Polysaccharid und 70 bis 50 Gew.-% Vinylpolymer.

Die Suspensionshilfsmittelkombination hat zwei Bestandteile:

1. Mindestens ein nichtionisches, wasserunlösliches und in dem aliphatischen Kohlenwasserstoff mindestens teilweise lösliches Tensid mit einem HLB-Wert kleiner als 10, vorzugsweise von 5 bis 10. Derartige Tenside sind vorzugsweise lipophile Sorbitanester wie zum Beispiel Sorbitanmonolaurat (HLB-Wert = 8,6) oder Sorbitanmonopalmitat (HLB-Wert = 6,7). Außerdem sind Polyethylenglykol(200)-monooleat (HLB-Wert = 7,0 bis 8,3), Polyethylenglykol(200)-monolaurat (HLB-Wert = 9,6) oder Polyethylenglykol-(300)-oleat (HLB-Wert = 8,9) gut geeignet. Weniger wirksam sind Sorbitanmonooleat oder -stearat (HLB-Wert = 4,3 bzw. 4,7). Diese lipophilen Tenside werden in Konzentrationen von 1 bis 10 Gew.-%, bezogen auf das wasserlösliche Monomer, eingesetzt. Vorzugsweise liegt die Tensidkonzentration bei 2 bis 5 Gew.-%.

2. Mindestens ein nichtionisches, wasserlösliches, Polyethylenglykolgruppen enthaltendes Tensid mit einem HLB-Wert größer als 10, vorzugsweise von 12 bis 20. Derartige Tenside sind beispielsweise Polyethylenglykolether aus einem aliphatischen einwertigen Alkohol mit 6 bis 20 C-Atomen und einem

4

Polyethylenglykol mit 3 bis 30, insbesondere 4 bis 20 Ethylenoxideinheiten. Geeignet sind handels übliche $C_{12}$-Fettalkoholpolyglykolether mit 7 bis 19 Ethylenoxideinheiten und einem HLB-Wert von 13 bis 18. Besonders geeignet sind wasserlösliche Polyethylenglykole mit einem Molekulargewicht von 200 bis 20 000, insbesondere 400 bis 5 000. Geeignet sind ferner Polyoxyethylen-Sorbitanfettsäureester mit einem HLB-Wert von 10 bis 20 wie Polyoxyethylen-Sorbitan-monolaurat (HLB-Wert 16,7), Polyoxyethylen-Sorbitan-monopalmitat (HLB-Wert 15,6) oder Polyoxyethylen-Sorbitan-monooleat (HLB-Wert 15,0). Diese Zusatzstoffe werden in Konzentrationen von 1 bis 10 Gew.-%, bezogen auf das wasserlösliche Monomer, eingesetzt. Vorzugsweise beträgt die Konzentration 2 bis 5 Gew.-%.

Die Pfropfpolymerisation wird durch Zugabe eines wasserlöslichen, freie Radikale liefernden Initiators wie Kaliumpersulfat, Natriumpersulfat und/oder Ammoniumpersulfat und durch Erhitzen auf 40 bis 100 °C ausgelöst. Weitere geeignete Initiatoren sind Hydroperoxide wie Wasserstoffperoxid, tert.-Butylhydroperoxid oder Cumolhydroperoxid. Die Peroxide sind im Temperaturbereich von 50 bis 80 °C wirksam. Sie können auch zusammen mit wasserlöslichen Reduktionsmitteln wie Ascorbinsäure und/oder Fe(II)-Salzen, Natriumbisulfit oder Natriumformaldehydsulfoxylat als Redox-Paar zur Initiierung bei tieferen Temperaturen von 20 bis 50 °C verwendet werden. Die Konzentration der Peroxide beträgt, bezogen auf das wasserslösliche Monomer, 0,05 bis 2 Gew.-%, vorzugsweise 0,2 bis 0,5 Gew.-%. Die reduzierenden Komponenten können in Konzentrationen bis zu 0,2 Gew.-%, bezogen auf das Monomer, eingesetzt werden.

Während oder nach der Polymerisation können 0,01 bis 2 Gew.-% eines vollständig oder überwiegend wasserlöslichen Vernetzungsmittels zugesetzt werden. Geeignet sind Vinylverbindungen wie N'N-Methylenbisacrylamid, Butandiol-1.4-di(meth)acrylat, Neopentylglykoldimethacrylat, Ethandioldi(meth)acrylat, Dialkylmaleinat, Glycidylmethacrylat, Allylmethacrylat, Polyethylenglykol(450)dimethacrylat oder Ethylenglykoldiglycidylether.

Die Pfropfpolymerisation wird in üblichen, für diskontinuierliche Reaktionsführung geeigneten Reaktionsbehältern durchgeführt, die mit einem Rührer ausgerüstet sind und wahlweise geheizt und gekühlt werden können. Die Pfropfpolymerisation wird in der Weise gestartet, daß die Ausgangsmischung unter Stickstoff auf die Reaktionstemperatur aufgeheizt und danach der Initiator zugesetzt wird. Es kann jedoch auch so verfahren werden, daß der Initiator zum Polymerisationsansatz zugegeben wird und die Polymerisation unter Inertgas durch Erwärmen gestartet wird. Beide Initiierungsmethoden führen zu hochwertigen Produkten.

Die Polymerisation wird vorzugsweise bei 40 bis 80 °C durchgeführt. Besonders bevorzugt sind Temperaturen von 55 bis 65 °C. Die Reaktionszeit beträgt 0,5 bis 6 Stunden. Da die Pfropfpolymerisation eine exotherme Reaktion ist, wird während der Reaktion gekühlt. Es ist jedoch nicht nötig, die Polymerisation unter isothermen Bedingungen durchzuführen.

Die Produkte fallen als feinteilige Körner oder Perlen an. Sie können leicht von der kontinuierlichen Phase getrennt und nach üblichen Verfahren, beispielsweise unter Vakuum oder unter Anwendung eines Wirbelschichttrockners, zu pulverförmigem Produkt getrocknet werden. Das Filtrat kann in der nächsten Polymerisationscharge wiederverwendet werden. Im Anschluß an die Polymerisation kann das Wasser auch durch azeotrope Destillation entfernt werden.

Als feinteilige Produkte werden im Sinne dieser Erfindung Produkte mit Korngrößen unter 2 mm verstanden.

Die Bildung von grobteiligen Agglomeraten und Anbackungen ist sehr gering.

Die Kombination aus einem lipophilen und hydrophilen Tensid ist ein wesentlicher Bestandteil der vorliegenden Erfindung: Sie ermöglicht einerseis eine homogene verklumpungsfreie Verteilung der Stärkekörner in der wäßrigen Monomerphase und andererseits eine sehr gute Zerteilung der Monomerphase zusammen mit den Stärkepartikeln zu feinen Tröpfchen in der organischen Phase.

Es wird überraschenderweise eine Verkleisterung zu einem zusammenhängenden, hochviskosen und - schlecht rührfähigen Gel vermieden, obwohl sonst erfahrungsgemäß bei der Erwärmung von Stärke in wäßriger salzhaltiger Lösung oder in Gegenwart von wäßrigem Alkali Verkleisterung auftritt. Die erfindungsgemäße Tensidkombination ermöglicht also eine glatte, störungsfreie Pfropfpolymerisation in diskontinuierlicher Fahrweise.

Bei einsetzender Polymerisation tritt keine vorübergehende oder bleibende Gel-oder Klumpenbildung auf. Der Anteil an polymeren Abscheidungen an Kesselwand und Rührer ist deutlich vermindert gegenüber der Pfropfpolymerisation in Gegenwart von nur lipophilen Tensiden.

Zusammenfassend hat das erfindunsgemäße Verfahren folgende Vorteile:

1. Das einstufig und diskontinuierlich durchgeführte inverse Suspensionspolymerisationsverfahren erfordert erheblich geringeren apparativen Aufwand als die bekannten halbkontinuierlichen Verfahren, da die gesonderte Bereitung und Dosierung der wäßrigen Monomerlösung entfällt.

2. Die Pfropfpolymerisation zu feinteiligem Produkt ist verbessert. Die Neigung zur Gel-und Klumpenbildung ist verringert.

3. Das Endprodukt zeichnet sich durch eine erhöhte Wasserabsorptionskapazität aus.
Die folgenden Beispiele sollen das Verfahren verdeutlichen.

Vergleichsbeispiel A

Nach den Angaben der japanischen Patentschrift 80-161 813 werden in einem 2 l-Vierhals-Glaskolben, der mit Rührer, Rückflußkühler, Thermometer, Tropftrichter und Stickstoff-Einleitung ausgerüstet ist, 450 ml Cyclohexan, 78 g Acrylsäure unter Rühren (400 UpM) vermischt. Um Acrylsäure zu 75 % zu neutralisieren, gibt man nun unter Kühlung 153 g 22,6 %ige Natronlauge und dann 11,3 g native Maisstärke und 2,5 g Sorbitanmonostearat (DISPONIL$^R$SMS von Henkel, D-4000 Düsseldorf) zu. Der Ansatz wird 30 Minuten bei 58 °C gerührt. Danach werden 0,25 g Ammoniumpersulfat, gelöst in 10 ml Wasser, über einen Zeitraum von einer halben Stunde zugegeben. Die Polymerisation setzt mit einer Temperaturerhöhung von 5 °C ein, wobei sich teils grobteiliges, teils klumpenförmiges Pfropfpolymerisat bildet. Durch weiteres intensives Rühren werden die Klumpen zu groben Perlen zerteilt. Die Polymerisation wird 3 Stunden bei 60 °C fortgesetzt. Danach wird das Produkt durch Filtration von der organischen Phase abgetrennt und dann bei 50 °C in 24 Stunden im Wasserstrahlvakuum getrocknet.
Ausbeute:

106 g Produkt (85 % der theoretischen Ausbeute), davon
87 g (82 %) perlförmiges Produkt und
19 g (18 %) grobteilige Anbackungen.

Vergleichsbeipiel B

Es wird wie in Vergleichsbeispiel A verfahren. Es werden jedoch 43 g native Maisstärke eingesetzt (wodurch ein Pfropfpolymerisat mit einem Stärkeanteil von 30 Gew.-% erhalten wird). Die Pfropfpolymerisation wird durch Zugabe von 0,5 g Ammoniumsulfat, gelöst in 10 ml Wasser, initiiert.
Ausbeute:

143 g Produkt (92 % der theoretischen Ausbeute), davon
136 g (95 %) perlförmiges Produkt und     7 g (5 %) grobteilige Anbackungen.

Vergleichsbeispiel C

Es wird wie in Vergleichsbeispiel A verfahren. Es werden jedoch 100 g native Maisstärke eingesetzt (wodurch ein Pfropfpolymerisat mit einem Stärkeanteil von 50 Gew.-% erhalten wird). Nach Zugabe von 0,5 g Ammoniumsulfat, gelöst in 10 ml Wasser, und Erwärmung auf 58 °C beginnt die Polymerisation, wobei sich ein hochviskoses Reaktionsprodukt von puddingähnlicher Konsistenz bildet. Durch Zugabe von 30 ml Wasser und 50 ml Cyclohexan wird die Rührfähigkeit verbessert. Die Polymerisation wird nach 3 Stunden bei 60 °C beendet.
Ausbeute:

198 g Produkt (93 % der theoretischen Ausbeute), davon
181 g (91 %) perlförmiges Produkt und
17 g (9 %) grobteilige Anbackungen.

Beispiel 1

Es wird wie in Vergleichsbeispiel A verfahren. Es werden jedoch zusammen mit 2,5 g Sorbitanmono-stearat 2,5 g $C_{12}$-Fettalkoholpolyethylenglykolether mit 17 Ethylenoxid-Einheiten (MARLIPAL[R] 1217 von Hüls AG, D-4370 Marl) als zusätzliches nichtionisches Tensid eingesetzt. Die Polymerisation setzt mit einer Temperaturerhöhung von 5 °C ein, wobei sich das Pfropfpolymerisat in Form feiner Teilchen bildet. Ausbeute:

123 g Produkt (99 % der theoretischen Ausbeute), davon
121 g (98,4 %) feinteiliges Produkt und
2 g (1,6 %) grobteiliges Gel.

Beispiel 2

Es wird wie in Vergleichsbeispiel B ein Pfropfpolymer mit einem Stärkegehalt von 30 % hergestellt. Dabei wird jedoch die Tensidkombination von Beispiel 1 eingesetzt. Ausbeute:

157 g Produkt (99 % der theoretischen Ausbeute), davon
156 g (99,4 %) perlförmige Fraktion und
1 g (0,6 %) grobteiliges Gel, Anbackungen.

Beispiel 3

Es wird wie in Vergleichsbeispiel C ein Pfropfmischpolymer mit einem Stärkegehalt von 50 % hergestellt. Dabei wird jedoch die Tensidkombination von Beispiel 1 eingesetzt. Ausbeute:

219 g Produkt (100 % der theoretischen Ausbeute), davon
218 g (99,5 %) feinteilige Fraktion und
1 g (0,5 %) grobteiliges Gel.

Vergleichsbeispiel D

Es werden die gleichen Einsatzstoffe und Mengen wie in Vergleichsbeispiel B verwendet. Statt Sorbitanmonostearat werden jedoch 2,5 g Sorbitanmonolaurat (SPAN[R] 20 von Atlas, Wilmington, Del., USA) zugesetzt. Der Ansatz wird 30 Minuten bei 60 °C unter Überleiten von Stickstoff gerührt. Dann wird eine Lösung von 0,5 g Ammoniumpersulfat in 10 ml Wasser in einem Zeitraum von einer halben Stunde zugetropft und die Polymerisation eingeleitet. Es entsteht perlförmiges Pfropfpolymer, da abgesaugt und dann bei 50 °C in 24 Stunden vakuumgetrocknet wird. Ausbeute:

151 g Produkt (95 % der theoretischen Ausbeute), davon
120 g (79 %) perlförmiges Produkt und
31 g (21 %) grobteilige Anbackungen.

### Beispiel 4

Es wird wie in Vergleichsbeispiel D verfahren. Es werden jedoch neben 2,5 g Sorbitanmonolaurat 3 g Polyethylenglykol mit dem Molekulargewicht 1 550 (POLYDIOL 1550 von Hüls AG) eingesetzt. Bei einsetzender Polymerisation bildet sich feinteiliges perlförmiges Produkt.
Ausbeute:

156 g Produkt (97 % der theoretischen Ausbeute), davon
155 g (99,4 %) feinteilige, lockere Perlen und
1 g (0,6 %) grobteiliges Gel.

### Beispiel 5

Es wird wie in Beispiel 4 verfahren, mit dem Unterschied, daß die Polymerisation durch Zugabe von 1 g Ammoniumpersulfat, gelöst in 10 ml Wasser, gestartet wird.
Ausbeute:

157 g feinteiliges Produkt (97 % der theoretischen Ausbeute), ohne gelartige Abscheidungen.

### Beispiel 6

Dieses Beispiel beschreibt eine bevorzugte Ausführungsform im Technikumsmaßstab.

In einem 60 l-Polymerisationsautoklav aus $V_2$A-Stahl, ausgerüstet mit Blattrührer, Thermometer und Einfüllstutzen werden 30 l Leichtbenzin (EXSOL[R] von Esso AG) mit dem Siedepunktbereich 80 bis 110 °C vorgelegt. Dazu werden unter Rühren (180 UpM) 5,07 kg wasserfreie Acrylsäure und danach 9,36 kg 22,6 %ige Natronlauge langsam unter Kühlung des Kessels so zugegeben, daß die Innentemperatur 30 °C nicht übersteigt. Danach werden 162 g Sorbitanmonolaurat, 81 g Polyethylenglykol mit dem Molekulargewicht 1 550, 2,8 kg native Maisstärke sowie 32 g Kaliumperoxidisulfat zugegeben. Unter Stickstoff werden nun die Drehzahl auf 200 UpM und die Kesselinnentemperatur auf 55 °C erhöht. Die Pfropfpolymerisation springt nach 30 Minuten unter Temperaturerhöhung an. Am Rührerleistungsschreiber wird nur eine geringe Erhöhung der Stromaufnahme als Anzeichen eines glatten Polymerisationsverlaufs registriert. Die Polymerisation wird noch 3 Stunden fortgesetzt. Nach Abkühlung wird das Produkt, das in Form feiner lockerer poröser Gelteilchen anfällt, durch Abnutschen von der Benzinphase getrennt und getrocknet.
Ausbeute:

9,56 kg Produkt (95 % der theoretischen Ausbeute), davon
9,04 kg (94,6 %) perlförmiges Produkt und
0,52 kg (5,4 %) grobteilige Anbackungen an Rührer und Kessel.

### Beispiel 7

In einem 2 l-Vierhalskolben werden 450 ml Leichtbenzin mit einem Siedepunktbereich von 80 bis 110 °C vorgelegt und 63 g Acrylsäure darin verrührt. Es werden dann bei laufendem Rührer 115 g 22,6 %ige Natronlauge, 19 g Acrylamid, 43 g Maisstärke, 2,5 g Sorbitanmonolaurat, 2 g Polyethylenglykol mit einem Molekulargewicht von 1 550, 0,5 g Polyethylenglykol mit einem Molekulargewicht von $6 \times 10^6$ sowie 0,5 g Ammoniumpersulfat, gelöst in 10 ml Wasser, zugesetzt. Das Gemisch wird 15 Minuten mit 400 UpM gerührt und dabei auf 60 °C aufgeheizt. Nach 15 Minuten setzt die Polymerisation unter Viskositätserhöhung, aber ohne Verklumpung, ein. Nach dreistündiger Polymerisation wird das Polymerisat abfiltriert und bei 50 °C getrocknet.
Ausbeute:

154 g Produkt, davon
153 g (99,4 %) feinteiliges Produkt und
1 g (0,6 %) grobe Anbackungen.

Das Produkt hat eine Wasseraufnahmekapazität von 902 g VE-Wasser/g.

Man kann die Entwässerung und die Aufarbeitung auch in der Weise durchführen, daß man das Wasser nach der Polymerisation azeotrop bei 100 °C destilliert, wobei 71 g Wasser (80 %) abgetrennt werden. Das feinkörnige Produkt wird dann 24 Stunden bei 50 °C getrocknet. Die Wasseraufnahmekapazität beträgt nun 530 g VE-Wasser/g.

## Vergleichsbeispiel E

Dieses Vergleichsbeispiel zeigt die Polymerisation von Natriumacrylat in Gegenwart von Stärke (50 % im Polymerisat) ohne Verwendung eines Suspensionsmittels.

In einem 2 l-Vierhalskolben werden 550 ml Cyclohexan vorgelegt. Danach werden bei laufendem Rührer 78 g Acrylsäure, 144 g 22,6 %ige Natronlauge unter Kühlung sowie 100 g native Maisstärke zugesetzt. Unter Stickstoff wird die Temperatur auf 60 °C erhöht und dann 30 Minuten gehalten, wobei ein puddingähnliches Ausgangsgemisch entsteht. Nun wird die Polymerisation durch Zugabe von 0,5 g Kaliumpersulfat, gelöst in 10 ml Wasser, eingeleitet. Es bildet sich grobteiliges Polymerisat, das filtriert, bei 50 °C im Vakuum getrocknet und dann gemahlen wird.
Ausbeute:

137 g (76 %) gemahlenes Pulver und
43 g (24 %) klumpenartige Anbackungen an Rührer und Kolbenwand.

## Beispiel 8

Es wird wie im Vergleichsbeispiel E verfahren. Es werden jedoch zusätzlich 2,5 g Sorbitanmonolaurat und 5 g Polyethylenglykol mit dem Molekulargewicht 1 550 eingesetzt. Bei der Polymerisation entsteht überwiegend feinkörniges Produkt, mit einer
Ausbeute:

211 g feinteiliges Produkt (97 % der theoretischen Ausbeute).

Es treten keine gelartigen Anbackungen auf.

## Vergleichsbeispiel F

In einem 2 l-Dreihalskolben werden in 450 ml Leichtbenzin mit einem Siedebereich von 80 bis 110 °C bei laufendem Rührer 78 g Acrylsäure, 144 g 22,6 %ige Natronlauge, 2,5 g Sorbitanmonolaurat und 43 g native 10 ml Wasser, zugesetzt, worauf unter Stickstoff auf 55 °C erhitzt wird. Bei einsetzender Polymerisation entsteht verklumptes Produkt. Dieses wird isoliert, getrocknet und gemahlen.

## Vergleichsbeispiel G

Es wird versucht, das in DE-PS 28 40 010 beschriebene halbkontinuierliche Pfropfpolymerisationsverfahren mit gesonderter Bereitung und Dosierung der Monomerlösung, wobei eine Tensidkombination aus einem lipophilen Tensid (Sorbitanmonooleat) und einem hydrophilen, ionischen Tensid (Alkylbenzolsulfonat) eingesetzt wurde, auf das erfindungsgemäße, einstufige Polymerisationsverfahren zu übertragen. Dabei wird die in DE-PS 28 40 010 angegebene Zugabereihenfolge beibehalten.

Versuchsdurchführung: In 450 ml Leichtbenzin mit einem Siedebereich von 80 bis 110 °C werden nacheinander 43 g native Maisstärke, 2,5 g Sorbitanmonooleat, 3 g Natriumdodecylbenzolsulfonat (MARLON[R] A 350 von Hüls AG), 144 g 22,6 %ige Natronlauge und - unter Zutropfen - 78 g Acrylsäure zugesetzt und verrührt. Dann werden 0,5 g Ammoniumpersulfat, gelöst in 10 ml Wasser, zugesetzt und der Ansatz unter Stickstoffspülung auf 55 °C aufgeheizt. Bei einsetzender Polymerisation bildet sich das Produkt in Form eines Gelklumpens.

Wird die Reihenfolge der Zugabe der Komponenten insoweit geändert, als zuerst Acrylsäure und danach Natronlauge zugegeben werden, ist das Versuchsergebnis ebenfalls unbefriedigend, da auch hier ein Gelklumpen entsteht. Die verwendete Tensidkombination versagt also bei der in-situ-Bereitung der wäßrigen Monomerlösung und kann deshalb nicht auf das erfindungsgemäße Verfahren übertragen werden.

In Tabelle 1 sind die Ergebnisse der Beispiele 1 bis 8 und der Vergleichsbeispiele A bis G zusammengestellt. Die angegebenen Wasseraufnahmekapazitäten (Zentrifugenmethode) der Pfropfpolymere wurden wie folgt bestimmt:

Etwa 50 mg des getrockneten Produkts ($G_1$) mit einer durch Siebung erhaltenen Korngröße von 0 bis 200 m werden in einem 100 ml-Zentrifugenglas mit 80 ml VE-Wasser eine Stunde bei schwacher Rührung auf dem Magnetrührer gequollen. Der Gelanteil wird durch halbstündiges Zentrifugieren bei 5 000 UpM abgetrennt, durch Abtupfen mit Zellstoff von anhaftendem Sol befreit und dann gewogen ($G_2$). Wasseraufnahmekapazität:

$$\frac{G_2 - G_1}{G_1} \quad \text{(g VE-Wasser/g Produkt)}$$

Tabelle 1

| Beisp. | Einsatzstoffe | | | | | | Polymerisations-verlauf | Polymer | | Wasser-aufnahme-Kapazität |
|---|---|---|---|---|---|---|---|---|---|---|
| | Acryl-säure (g) | Neutr.-grad (%) | Acryl-amid (g) | Stärke (g) | lipohiles Tensid (g) | hydrophiles Tensid (g) | | fein-teilig (%) | grob-teilig (%) | (g/g) |
| A | 78 | 75 | | 11,3 | (1) 2,5 | - | Klumpenbildung | 82 | 18 | 451 |
| B | 78 | 75 | | 43 | (1) 2,5 | - | feinteilig | 95 | 5 | 403 |
| C | 78 | 75 | | 100 | (1) 2,5 | - | feinteilig | 91 | 9 | 372 |
| D | 78 | 75 | | 43 | (2) 2,5 | - | grobteilig | 79 | 21 | 475 |
| E | 78 | 70 | | 100 | - 2,5 | - | grobteilig | 76 | 24 | 210 |
| F | 78 | 70 | | 43 | (3) 2,5 | - | verklumpt | | 100 | 479 |
| G | 78 | 70 | | 43 | (3) 2,5 | (4) 2,5 | verklumpt | | 100 | 756 |
| 1 | 78 | 75 | | 11,3 | (1) 2,5 | (5) 2,5 | feinteilig | 98,4 | 1,6 | 488 |
| 2 | 78 | 75 | | 43 | (1) 2,5 | (5) 2,5 | feinteilig | 99,4 | 0,6 | 408 |
| 3 | 78 | 75 | | 100 | (1) 2,5 | (5) 2,5 | feinteilig | 99,5 | 0,5 | 388 |
| 4 | 78 | 75 | | 43 | (2) 2,5 | (6) 2,5 | feinteilig | 99,4 | 0,6 | 756 |
| 5 | 78 | 75 | | 43 | (2) 2,5 | (6) 2,5 | feinteilig | 100 | 0 | 826 |
| 6 | 5 070 | 75 | | 2 800 | (2) 162 | (6) 81 | feinteilig | 94,6 | 5,4 | 825 |
| 7 | 63 | 75 | 19 | 43 | (2) 2,5 | (6) 2,5 | feinteilig | 99,3 | 0,6 | 902 |
| 8 | 78 | 70 | - | 100 | (2) 2,5 | (6) 5,0 | feinteilig | 100 | - | 530 |

(1) Sorbitanmonostearat  (3) Sorbitanmonooleat  (5) $C_{12}$-Fettalkoholpolyethylenglykolether

(2) Sorbitanmonolaurat  (4) Natriumdodecylbenzolsulfonat  (6) Polyethylenglykol mit einem Molekular-gewicht von 1 550

0 242 529

## Ansprüche

1. Verfahren zur Herstellung von stark wasserquellbaren, teilchenförmigen Pfropfpolymen aus einem Polysaccharid und mindestens einem wasserlöslichen Carboxyl-oder Carboxylatgruppen enthaltenden Vinylmonomeren,
dadurch gekennzeichnet,
daß man in einer Dispersion aus

    a) einem aliphatischen Kohlenwasserstoff

    b) einer wäßrigen Lösung mindestens eines wasserlöslichen, ethylenisch ungesättigten Monomeren.

    c) einem Polysaccharid und

    d) einer Kombination aus

        (1) einem nichtionischen, wasserunlöslichen, in (a) mindestens teilweise löslichen Tensid mit einem Hydrophil-Lipophil-Gleichgewicht kleiner als 10 und

        (2) einem nichtionischen, wasserlöslichen, Polyethylenglykolgruppen enthaltenden Tensid mit einem Hydrophil-Lipophil-Gleichgewicht größer als 10

eine einstufige, diskontinuierliche, radikalische Polmerisation unter Erhitzen der Reaktionsmischung auf 40 bis 100 °C durchführt und nach der Reaktion das gebildete Pfropfpolymer als Pulver abtrennt.

2. Verfahren nach Anspruch 1,
dadurch gekennzeichnet,
daß man die Polymerisation in Gegenwart eines Tensids mit einem Hydrophil-Lipophil-Gleichgewicht von 5 bis 10 und eines Polyethylenglykolgruppen enthaltenden Tensids mit einem Hydrophil-Lipophil-Gleichgewicht von 12 bis 20 durchführt.

3. Verfahren nach den Ansprüchen 1 und 2,
dadurch gekennzeichnet,
daß man ein Pfropfpolymer aus 10 bis 70 Gew.-% Polysaccharid und 90 bis 30 Gew.-% Vinylpolymer herstellt.

4. Verfahren nach den Ansprüchen 1 bis 3,
dadurch gekennzeichnet,
daß man ein Pfropfpolymer aus 30 bis 50 Gew.-% Polysaccharid und 70 bis 50 Gew.-% Vinylpolymer herstellt.

5. Verfahren nach den Ansprüchen 1 bis 4,
dadurch gekennzeichnet,
daß als Polysaccharid Stärke verwendet wird.

6. Verfahren nach Anspruch 1,
dadurch gekennzeichnet,
daß man eine wäßrige Lösung von (Meth)Acrylsäure einsetzt und vor der Polymerisation die (Meth)-Acrylsäure in Gegenwart des aliphatischen Kohlenwasserstoffs zu 50 bis 95 % neutralisiert.

7. Verfahren nach den Ansprüchen 1 und 2,
dadurch gekennzeichnet,
daß das wasserunlösliche Tensid ein Sorbitanester und das wasserlösliche Tensid ein Alkyloxethylat oder Polyethylenglykol ist.

8. Verwendung von nach den Ansprüchen 1 bis 7 hergestellten Pfropfpolymeren als wasserabsorbie-rende Mittel.